(19) Europäisches Patentamt
European Patent Office
Office européen des brevets

(11) EP 0 979 818 B1

(12) **EUROPEAN PATENT SPECIFICATION**

(45) Date of publication and mention of the grant of the patent:
**19.02.2003 Bulletin 2003/08**

(21) Application number: **98907277.2**

(22) Date of filing: **16.03.1998**

(51) Int Cl.[7]: **C07D 211/26**, A61K 31/445

(86) International application number:
**PCT/JP98/01132**

(87) International publication number:
**WO 98/042668 (01.10.1998 Gazette 1998/39)**

(54) **BENZOIC ACID COMPOUNDS AND MEDICINAL USE THEREOF**

BENZOESÄUREDERIVATE UND DEREN MEDIZINISCHE VERWENDUNG

COMPOSES D'ACIDE BENZOIQUE ET LEUR UTILISATION MEDICALE

(84) Designated Contracting States:
**AT BE CH DE DK ES FI FR GB GR IE IT LI LU MC NL PT SE**

(30) Priority: **21.03.1997 JP 6873997**

(43) Date of publication of application:
**16.02.2000 Bulletin 2000/07**

(73) Proprietor: **Mitsubishi Pharma Corporation**
**Osaka-shi, Osaka 541-0046 (JP)**

(72) Inventors:
- **ITO, Katsuhiko; Yoshitomi Pharm. Ind., Ltd. Chikujo-gun; Fukuoka 871-8550 (JP)**
- **SONDA, Shuji; Yoshitomi Pharm. Ind., Ltd. Hirakata-shi; Osaka 573-1153 (JP)**
- **KAWAHARA, Toshio; Yoshitomi Pharm. Ind. Ltd. Chikujo-gun; Fukuoka 871-8550 (JP)**
- **ASANO, Kiyoshi; Yoshitomi Pharm. Ind., Ltd. Chikujo-gun;Fukuoka 871-8550 (JP)**
- **KAWAKITA, Takeshi; Yoshitomi Pharm. Ind. Ltd. Chikujo-gun; Fukuoka 871-8550 (JP)**

(74) Representative:
**von Kreisler, Alek, Dipl.-Chem. et al**
**Patentanwälte,**
**von Kreisler-Selting-Werner,**
**Bahnhofsvorplatz 1 (Deichmannhaus)**
**50667 Köln (DE)**

(56) References cited:

| | |
|---|---|
| **EP-A- 0 774 460** | **EP-A- 0 873 990** |
| **WO-A-95/26953** | **WO-A-97/11054** |
| **JP-A- 9 077 742** | **JP-A- 9 241 241** |

- **M S HADLEY ET AL: "Substituted Benzamides with Conformationally Restricted Side Chains. 3. Azabicyclo[x.y.0] Derivatives as Gastric Prokinetic Agents" BIOORGANIC & MEDICINAL CHEMISTRY LETTERS,GB,OXFORD, vol. 2, no. 2, 1 January 1992 (1992-01-01), pages 1147-1152, XP002077997 ISSN: 0960-894X**
- **GASTER L M ET AL: "(1-BUTYL-4-PIPERIDINYL)METHYL 8-AMINO-7-CHLORO-1,4-BENZODIOXANE-5-CA R BOXYLATE HYDROCHLORIDE: A HIGHLY POTENT AND SELECTIVE 5-HT4 RECEPTOR ANTAGONIST DERIVED FROM METACLOPRAMIDE" JOURNAL OF MEDICINAL CHEMISTRY,US,AMERICAN CHEMICAL SOCIETY. WASHINGTON, vol. 36, 1 January 1993 (1993-01-01), pages 4121-4123, XP000196066 ISSN: 0022-2623**
- **IWANAMI S ET AL: "SYNTHESIS AND NEUROLEPTIC ACTIVITY OF BENZAMIDES CIS-N-(1-BENZYL-2-METHYLPYRROLIDIN-3-YL) -5 -CHLORO-2-METHOXY-4- (METHYLAMINO)BENZAMIDE AND RELATED COMPOUNDS" JOURNAL OF MEDICINAL CHEMISTRY,US,AMERICAN CHEMICAL SOCIETY. WASHINGTON, vol. 24, no. 10, 1 October 1981 (1981-10-01), pages 1224-1230, XP000611487 ISSN: 0022-2623**

EP 0 979 818 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

• **H HARADA ET AL: "Development of Potent Serotonin-3 (5HT(3)) Recetor Antagonists. I. Structure-Activity Relationships of 2-Alkoxy-4-amino-5-chlorbenzamide Derivatives" CHEMICAL AND PHARMACEUTICAL BULLETIN,JP,PHARMACEUTICAL SOCIETY OF JAPAN. TOKYO, vol. 8, no. 43, 1 January 1995 (1995-01-01), pages 1364-1378, XP002077996 ISSN: 0009-2363**

Remarks:

The file contains technical information submitted after the application was filed and not included in this specification

## Description

Technical Field

The present invention relates to novel benzoic acid compounds. More particularly, the present invention relates to novel benzoic acid compounds having high and selective affinity for serotonin 4 (hereinafter to be referred to as 5-$HT_4$) receptors and capable of activating them, which are useful for the prophylaxis and therapy of various gastrointestinal diseases, central nervous system disorders, cardiac function disorders, urinary diseases and the like, pharmaceutically acceptable salts thereof, and use thereof as medicaments.

Background Art

When control mechanism of gastrointestinal motility fails and prokinetic function is declined, atonic constipation and digestive symptoms such as abdominal distention, anorexia and heartburn emerge. Declined gastrointestinal motility is found with aging, stress or diseases such as chronic gastritis, non-ulcer dyspepsia, reflux esophagitis, peptic ulcer, diabetes and the like, and gastrointestinal prokinetic agents have been used for treatment.

Ever since metoclopramide (The Merck Index, 11th edition, 6063) was developed as a gastrointestinal prokinetic agent, various substituted benzamide derivatives have been synthesized. At present, cisapride (The Merck Index, 11th edition, 2318) and others have been clinically used as gastrointestinal prokinetic agents besides metoclopramide. While benzamide derivatives such as metoclopramide and cisapride have been speculated to show effects via certain receptors in the digestive organs, the actual function of the receptors involved in the promotion of gastrointestinal motility has long been unclarified. Recently, however, 5-$HT_4$ receptor has been identified to be a new serotonin receptor subtype which stimulates adenylate cyclase activity, and benzamide derivatives have been found to promote gastrointestinal motility by activating 5-$HT_4$ receptor in the digestive organs [The Journal of Pharmacology and Experimental Therapeutics, vol. 252, pp. 1378-1386 (1990), European Journal of Pharmacology, vol. 196, pp. 149-155 (1991)]. Having found the action of metoclopramide and cisapride as 5-$HT_4$ receptor agonists, many attempts have been made to use 5-$HT_4$ receptor agonists as gastrointestinal prokinetic agents. The Journal of Pharmacology and Experimental Therapeutics, vol. 264, pp. 240-248 (1993) reports that substituted benzamide (SC53116) which is a 5-$HT_4$ receptor agonist stimulated gastrointestinal motility. As 5-$HT_4$ receptor agonists, Japanese Patent Unexamined Publication No. 157518/1994 discloses oxadiazole derivatives; Japanese Patent Unexamined Publication No. 10881/1995 discloses oxazabicyclo derivatives; WO 94/12497 discloses endo-N-(8-methyl-8-azabicyclo[3.2.1]oct-3-yl)-1-isopropyl-2(1H)-quinolone-3-carboxamide and acid addition salts thereof; and WO95/26953 discloses benzoic acid compounds.

The 5-$HT_4$ receptor has been found to be also present in brain (e.g., prefrontal area, nigra, hippocampus and amygdaloid complex), heart, endocrine system and urinary system [British Journal of Pharmacology, vol. 109, pp. 618-624 (1993) and Naunyn-Schmiedeberg's Archives of Pharmacology, vol. 344, pp. 150-159 (1991), Trends in Pharmacological Sciences, vol. 13, pp. 141-145 (1992), Pharmacological Reviews, vol. 46, pp. 182-185 (1994)].

In addition, since activation of 5-$HT_4$ receptor in the central nervous system promotes release of acetylcholine in prefrontal area, 5-$HT_4$ receptor is responsible for memory and learning mechanism through release of acetylcholine. Therefore, a 5-$HT_4$ agonist has a potential for a drug for the prophylaxis and treatment of disturbance of memory, dementia and the like. In view of a report documenting that 5-$HT_4$ receptor on GABA neuron is involved in anxiety, it also has a potential for a drug for the prophylaxis and treatment of anxiety. The action of 5-$HT_4$ receptor on heart and urinary system has been reported [Trends in Pharmacological Sciences, vol. 16, pp. 391-398 (1995)].

In view of such disclosures, a compound having 5-$HT_4$ receptor activating action is considered to be clinically applicable to digestive organs, brain, heart and urinary system. In other words, a 5-$HT_4$ receptor agonist should be useful as a medicament for the prophylaxis and treatment of various gastrointestinal diseases (e.g., reflux esophagitis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, abdominal indefinite complaint, and the like; constipation such as atonic constipation, chronic constipation, and constipation caused by spinal cord injury, pelvic diaphragm failure and the like; and irritable bowel syndrome), central nervous system disorders (e.g., schizophrenia, depression, anxiety, disturbance of memory and dementia), cardiac function disorders (e.g., heart failure and myocardial ischemia), urinary diseases (e.g., dysuria caused by urinary obstruction, ureterolith, prostatomegaly, spinal cord injury, pelvic diaphragm failure, etc.) and the like.

In addition, a report has documented that 5-$HT_4$ receptor antagonist shows an antagonistic action on analgesic action of cisapride and metoclopramide [Arzneimittel Forschung, vol. 43, pp. 913-918 (1993)]. Thus, a 5-$HT_4$ receptor agonist is expected to be useful as an anti-nociceptor for analgesic use which increases threshold of pain.

While there have been documented some benzoic acid compounds which have N-alkyl substituted cyclic

aminoalkyl in the amide moiety, and which selectively activate 5-$HT_4$ receptors [Journal of Medicinal Chemistry, vol. 35, pp. 1486-1489 (1992)], a compound has not been known at all, which has sulfonyl at an alkyl moiety bound to nitrogen atom of cyclic amine. WO97/11054 discloses benzoic acid compounds as a 5-$HT_4$ receptor agonist.

The above-mentioned substituted benzamide derivatives, such as metoclopramide, have, besides 5-$HT_4$ receptor agonistic effect, dopamine $D_2$ (hereinafter referred to as $D_2$) receptor antagonism or serotonin 3 (hereinafter referred to as 5-$HT_3$) receptor antagonism, and are not entirely satisfactory in terms of efficacy and safety, since they cause side effects such as extrapyramidal disorders due to $D_2$ receptor antagonism, and side effects such as constipation due to 5-$HT_3$ receptor antagonism [Drugs, vol. 41, pp. 574-595 (1991)], and are associated with such problems to be solved.

The compounds disclosed in Japanese Patent Unexamined Publication Nos. 262724/1993, 50883/1989 and 211685/1992 have been investigated as gastrointestinal prokinetic agents, and it has been found that these compounds show not only 5-$HT_4$ receptor activating action but also 5-$HT_3$ receptor antagonism. Therefore, these compounds are also considered to cause side effects such as constipation due to the 5-$HT_3$ receptor antagonism, as mentioned above, and are not satisfactory.

The 5-$HT_4$ receptors have been reported to be widely distributed in the entire digestive tract, and a 5-$HT_4$ receptor agonist having low affinity for $D_2$ receptor and 5-$HT_3$ receptor, but having high and selective affinity for 5-$HT_4$ receptor is expected to make a superior gastrointestinal prokinetic agent.

The benzoic acid compound disclosed in WO95/26953 has more selective and higher affinity for 5-$HT_4$ receptor as compared to that for $D_2$ receptor and 5-$HT_3$ receptor, and activates 5-$HT_4$ receptor. However, it shows poor absorption by oral administration and low bioavailability. Thus, there is a need for the development of a compound which has selective and high affinity for 5-$HT_4$ receptor, which activates same and which is superior in absorption.

Accordingly, the present invention aims at providing a compound having high and selective affinity for 5-$HT_4$ receptors and capable of activating them in various tissues, which is useful as a medicament for the prophylaxis and treatment of various gastrointestinal diseases (e.g., reflux esophagitis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, abdominal indefinite complaint, and the like; constipation such as atonic constipation, chronic constipation, and constipation caused by spinal cord injury, pelvic diaphragm failure and the like; and irritable bowel syndrome), central nervous system disorders (e.g., schizophrenia, depression, anxiety, disturbance of memory and dementia), cardiac function disorders (e.g., heart failure and myocardial ischemia), urinary diseases (e.g., dysuria caused by urinary obstruction, ureterolith, prostatomegaly, spinal cord injury, pelvic diaphragm failure, etc.), and the like, and which shows superior absorption and less toxicity.

In view of the above situation, the present inventors have made intensive studies in an attempt to find a compound which has selective and high affinity for 5-$HT_4$ receptor, which is capable of activating same and which has superior absorption and less toxicity, and found that a new benzoic acid compound, namely, a benzoic acid compound having 3-(benzylsulfonyl)propyl at the nitrogen atom of piperidine shows superior, selective and high affinity for 5-$HT_4$ receptors, activates them and has superior absorption and less toxicity, which resulted in the completion of the invention.

Disclosure of the Invention

Thus, the present invention provides the following.

(1) 4-Amino-5-chloro-2-methoxy-N-((1-(3-benzylsulfonylpropyl)piperidin-4-yl)methyl)benzamide (hereinafter sometimes referred to as compound (I)), a pharmaceutically acceptable salt thereof or an N-oxide compound thereof.

(2) A pharmaceutical composition comprising 4-amino-5-chloro-2-methoxy-N-((1-(3-benzylsulfonylpropyl)piperidin-4-yl)methyl)benzamide, a pharmaceutically acceptable salt thereof or an N-oxide compound thereof, and a pharmaceutically acceptable additive.

(3) A serotonin 4 receptor agonist comprising 4-amino-5-chloro-2-methoxy-N-((1-(3-benzylsulfonylpropyl)piperidin-4-yl)methyl)benzamide, a pharmaceutically acceptable salt thereof or an N-oxide compound thereof as an active ingredient.

(4) A gastrointestinal prokinetic agent comprising 4-amino-5-chloro-2-methoxy-N-((1-(3-benzylsulfonylpropyl)piperidin-4-yl)methyl)benzamide, a pharmaceutically acceptable salt thereof or an N-oxide compound thereof as an active ingredient.

(5) A prophylactic or therapeutic agent for gastrointestinal diseases selected from reflux esophagitis; gastroesophageal reflux; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis and diabetes; gastrointestinal disorder which is indigestion, meteorism or abdominal indefinite complaint; constipation which is atonic constipation, chronic constipation, or constipation caused by spinal cord injury or pelvic diaphragm failure; and irritable bowel syndrome, which agent comprises 4-amino-5-chloro-2-methoxy-N-((1-(3-benzylsulfonylpropyl)piperidin-4-yl)methyl)benzamide, a pharmaceutically acceptable salt thereof or an N-oxide compound thereof as an active ingredient.

The compound of the present invention is characterized by substitution of 3-(benzylsulfonyl)propyl at the nitrogen atom of piperidine, and, with regard to the piperidine, the bonding group

$$-CONHCH_2-$$

is bonded to the carbon atom other than the carbon atoms adjacent to the nitrogen atom in the ring. Such characteristic chemical structure affords selective high affinity for 5-$HT_4$ receptor and activation thereof by the compound of the present invention. The compound of the present invention having 3-(benzylsulfonyl)propyl introduced into the nitrogen atom of piperidine characteristically shows superior absorption and less toxicity. In the present invention, "selective" means low affinity for $D_2$ receptors and 5-$HT_3$ receptors and high affinity for 5-$HT_4$ receptors.

The pharmaceutically acceptable salts of the compound of the present invention are, for example, acid addition salt and quaternary ammonium salt. Examples of the acid addition salt include, for example, hydrochloride, sulfate, hydrobromide, phosphate, nitrate, methanesulfonate, ethanesulfonate, fumarate, maleate, benzoate, citrate, malate, mandelate, p-toluenesulfonate, acetate, succinate, malonate, lactate, salicylate, gallate, picrate, carbonate, ascorbate, trifluoroacetate and tartrate. Examples of quaternary ammonium salt include, for example, quaternary ammonium salts with lower alkyl halide (e.g., methyl iodide, methyl bromide, ethyl iodide, ethyl bromide and the like), lower alkylsulfonate (e.g., methyl methanesulfonate, ethyl methanesulfonate and the like), and lower alkyl arylsulfonate (e.g., methyl p-toluenesulfonate and the like). The N-oxide compounds at piperidine of compound (I) is also encompassed in the compound of the present invention. The compound of the present invention may be hydrates (e.g., monohydrate, 1/2 hydrate and 3/2 hydrate) or solvates.

The compound of the present invention can be produced by the following methods.

Method 1: The compound (I) can be synthesized by the following route.

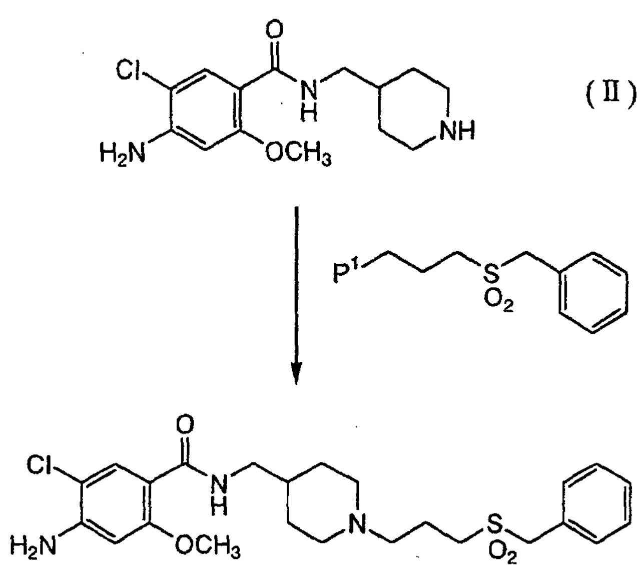

(II)

(III)

(I)

wherein $P^1$ is a leaving group such as halogen (e.g., chlorine, bromine, iodine, etc.) or sulfonyloxy (e.g., methanesulfonyloxy, p-toluenesulfonyloxy, trifluoromethanesulfonyloxy, etc.).

That is, compound (I) can be produced by reacting compound (II) with compound (III) in a suitable solvent in the presence of a base.

The solvent to be used for the reaction includes methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. The base to be used may be sodium carbonate, potassium carbonate, sodium hydrogencarbonate, potassium hydrogencarbonate, sodium hydroxide, potassium hydroxide, triethylamine, diisopropylethylamine, pyridine, sodium hydride, sodium methoxide or potassium tert-butoxide. While the reaction temperature varies depending on the kind of solvent to be used, it is generally from 0°C to 140°C, and the reaction time which varies depending on reaction temperature is generally 1-24 hours.

Method 2: The compound (II), which is an intermediate shown in Method 1, can be synthesized by the following route.

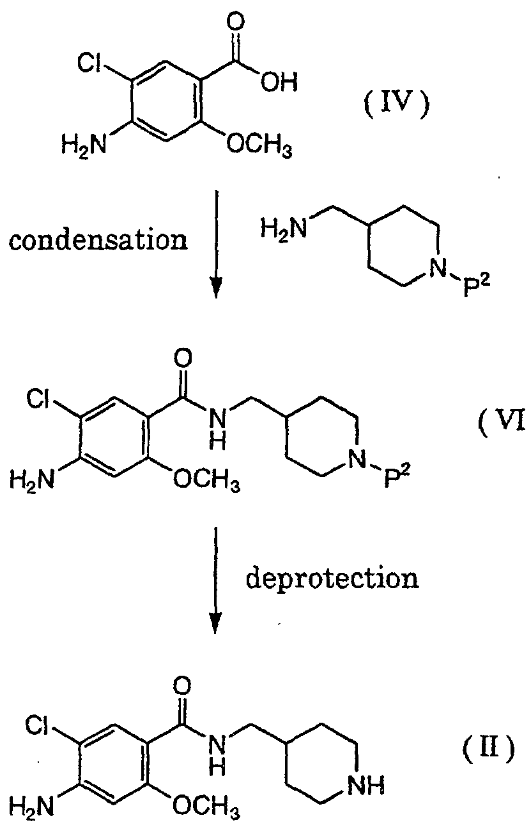


(IV) (V) (VI) (II)

wherein $P^2$ is a urethane type amino-protecting group such as tert-butoxycarbonyl, benzyloxycarbonyl, isopropyloxycarbonyl and the like.

That is, compound (II) can be produced by condensing a carboxylic acid represented by compound (IV) or its reactive derivative with compound (V) in a suitable solvent to give compound (VI) and deprotecting the compound (VI).

When compound (IV) is a free carboxylic acid, condensation is carried out using a routine condensing agent. Examples of the condensing agent include dicyclohexylcarbodiimide, 1-ethyl-3-(3-dimethylaminopropyl)carbodiimide hydrochloride, carbonyldiimidazole and N-methyl-2-chloropyridinium iodide. This reaction is preferably carried out in the presence of an organic base such as 1-hydroxybenzotriazole, N-methylmorpholine and the like. The solvent to be used includes dimethylformamide, dimethyl sulfoxide, methylene chloride, tetrahydrofuran, acetonitrile, dioxane and benzene. While the reaction temperature varies depending on the kind of solvent, it is generally from - 20°C to 50°C, and the reaction time which varies depending on reaction temperature is generally 1-24 hours.

When compound (IV) is a reactive derivative of carboxylic acid such as acid halide, acid anhydride, mixed acid anhydride and ester, condensation is generally carried out in a suitable solvent in the presence of a base as necessary. Examples of the base to be used as necessary include sodium carbonate, potassium carbonate, sodium hydrogencarbonate, potassium hydrogencarbonate, sodium hydroxide, potassium hydroxide, triethylamine, diisopropylethylamine and pyridine. The solvent to be used includes methylene chloride, chloroform, ethyl acetate, diethyl ether, tetrahydrofuran, dioxane, acetonitrile, benzene, toluene and mixed solvents thereof. While the reaction temperature varies depending on the kind of solvent, it is generally from -30°C to 50°C, and the reaction time which varies depending on reaction temperature is generally 1-24 hours.

The deprotection of compound (VI) can be carried out by a method generally used for removing amino-protecting group. For example, when $P^2$ is tert-butoxycarbonyl, the compound is treated with an acid in a suitable solvent as necessary. The acid to be used in this reaction may be any as long as amide bonding is not hydrolyzed, and may be, for example, hydrogen chloride, sulfuric acid, trifluoroacetic acid or trifluoromethanesulfonic acid. The solvent to be used as necessary includes methanol, ethanol, propanol, isopropyl alcohol, tetrahydrofuran, acetone, ethyl acetate, methylene chloride, chloroform, dioxane, water and mixed solvents thereof. The reaction temperature is from ice-cooling to refluxing temperature of solvent, preferably room temperature, and the reaction time is from 30 minutes to 5 hours.

Method 3: Compound (I) can be also synthesized by the following route.

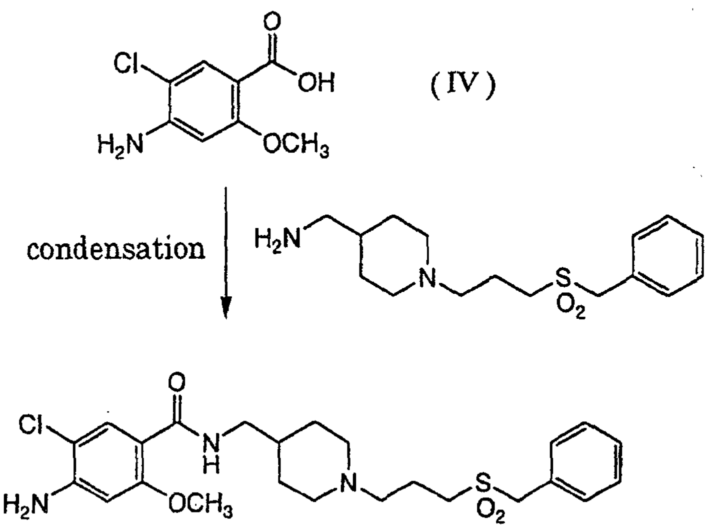


(IV)

(VII)

(I)

That is, the compound (I) can be produced by condensing carboxylic acid represented by compound (IV) or reactive derivative thereof with compound (VII) in a suitable solvent.

When compound (IV) is a free carboxylic acid, the condensation is carried out using a routine condensing agent. Examples of the condensing agent include dicyclohexylcarbodiimide, 1-ethyl-3-(3-dimethylaminopropyl)carbodiimide hydrochloride, carbonyldiimidazole, N-methyl-2-chloropyridinium iodide and the like. The instant reaction is preferably carried out by condensation in the presence of an organic base such as 1-hydroxybenzotriazole and N-methylmorpholine. The solvent to be used may be dimethylformamide, dimethyl sulfoxide, methylene chloride, tetrahydrofuran, acetonitrile, dioxane and benzene. While the reaction temperature varies depending on the kind of solvent to be used, it is generally from -20°C to 50°C, and the reaction time which varies depending on reaction temperature is generally 1-24 hours.

When compound (IV) is reactive derivative of carboxylic acid such as acid halide, acid anhydride, mixed acid anhydride and ester, the condensation is carried out in a suitable solvent in the presence of a base as necessary. The base to be used as necessary is sodium carbonate, potassium carbonate, sodium hydrogencarbonate, potassium hydrogencarbonate, sodium hydroxide, potassium hydroxide, triethylamine, diisopropylethylamine or pyridine. Examples of the solvent to be used include methylene chloride, chloroform, ethyl acetate, diethyl ether, tetrahydrofuran, dioxane, acetonitrile, benzene, toluene and mixed solvents thereof. While the reaction temperature varies depending on the kind of solvent to be used, it is generally from -30°C to 50°C, and the reaction time which varies depending on reaction temperature is generally 1-24 hours.

Method 4: Compound (VII), which is an intermediate shown in Method 3, can be synthesized by the following route.

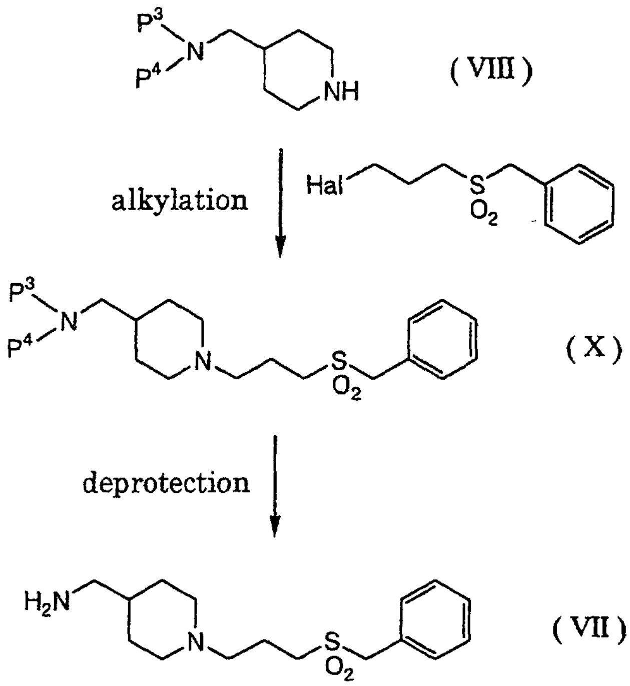


(VIII) (IX) (X) (VII)

wherein $P^3$ and $P^4$ are groups that form Schiff base together with adjacent nitrogen atom such as benzylidene, and Hal is halogen such as chlorine, bromine, iodine and the like.

That is, compound (VII) can be produced by subjecting compound (VIII) to alkylation with compound (IX) in a suitable solvent in the presence of a base to give compound (X) and deprotecting the compound (X).

The solvent to be used in the alkylation includes methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene, xylene and acetonitrile. The base to be used includes sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on reaction temperature is generally 1-24 hours.

The amino-deprotection reaction of compound (X) can be carried out by a method generally used for removing amino-protecting group. For example, the compound is treated with an acid in a suitable solvent. The acid to be used in this reaction includes, for example, hydrochloric acid, sulfuric acid, acetic acid, potassium hydrogensulfate, sulfamic acid, trifluoroacetic acid or methanesulfonic acid. The solvent to be used includes methanol, ethanol, propanol, isopropyl alcohol, acetonitrile, acetone, dioxane, toluene, dimethylformamide, dimethyl sulfoxide and mixed solvents thereof. The reaction temperature is from ice-cooling to 100°C, and the reaction time is from 30 minutes to 12 hours.

Compound (VIII) can be produced by reacting 4-aminomethylpiperidine with aldehyde or ketone, preferably aromatic aldehyde such as benzaldehyde. The instant reaction is carried out without solvent or in a suitable solvent at 0°C to 200°C for 1-24 hours. The suitable solvent to be used in the present reaction includes methanol, ethanol, propanol, isopropyl alcohol, acetonitrile, benzene, toluene, xylene, chloroform, methylene chloride, dichloroethane, dimethylformamide and dimethyl sulfoxide. In this reaction, the water to be formed can be removed with potassium hydroxide, or Dean-Stark water separator can be used to remove the water to be formed. The catalyst such as benzenesulfonic acid may be used in this reaction.

Method 5: Compound (III), which is an intermediate shown in Method 1, can be synthesized by the following route.

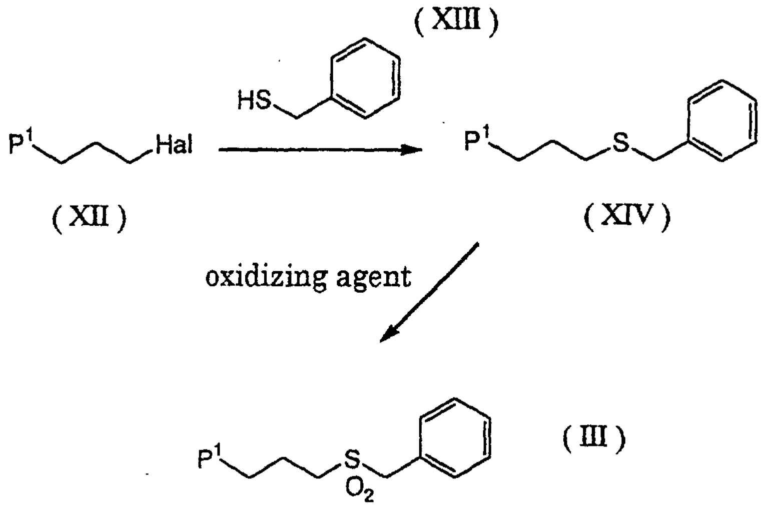


wherein each symbol is as defined above.

That is, compound (III) can be produced by reacting compound (XII) with compound (XIII) in a suitable solvent in the presence of a base to give compound (XIV) and oxidizing the compound using 2 equivalents of an oxidizing agent in the presence of an acid as necessary.

The solvent to be used for the reaction of compound (XII) and compound (XIII) includes methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the base to be used include sodium hydride, sodium carbonate, potassium carbonate, sodium hydrogencarbonate, potassium hydrogencarbonate, sodium hydroxide, potassium hydroxide, triethylamine, diisopropylethylamine and pyridine. While the reaction temperature varies depending on the kind of solvent to be used, it is generally from 0°C to 140°C, and the reaction time which varies depending on reaction temperature is generally 1-24 hours.

The oxidizing agent to be used for the oxidation includes ruthenium tetraoxide, chromic acid, permanganate, m-chloroperbenzoic acid and aqueous hydrogen peroxide solution. The solvent to be used for oxidation includes formic acid, acetic acid, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. Examples of the acid to be used as necessay include hydrochloric acid, sulfuric acid, p-toluenesulfonic acid and benzoic acid. While the reaction temperature varies depending on the kind of oxidizing agent to be used, it is generally from 0°C to 140°C, and the reaction time which varies depending on reaction temperature is generally 1-24 hours.

Method 6: Compound (I) can be also synthesized by the following route.

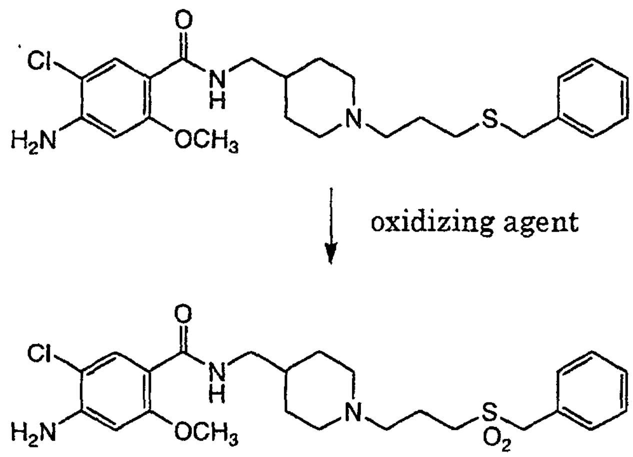


(XI)

(I)

That is, compound (I) can be produced by reacting compound (XI) with 2 equivalents of an oxidizing agent in a suitable solvent in the presence of an acid as necessary.

The oxidizing agent to be used for oxidation includes ruthenium tetraoxide, chromic acid, permanganate, m-chloroperbenzoic acid and aqueous hydrogen peroxide solution. The solvent to be used for oxidation includes formic acid, acetic acid, methylene chloride, 1,2-dichloroethane, chloroform, methanol, ethanol, propanol, isopropyl alcohol, butanol, tetrahydrofuran, diethyl ether, dioxane, dimethylformamide, dimethyl sulfoxide, benzene, toluene and xylene. The acid to be used as necessary includes hydrochloric acid, sulfuric acid, p-toluenesulfonic acid or benzoic acid. While the reaction temperature varies depending on the kind of oxidizing agent to be used, it is generally from 0°C to 140°C, and the reaction time which varies depending on reaction temperature is generally 1-24 hours.

Compound (XI) can be produced, for example, by conducting the reaction using compound (XIV) instead of compound (III) in Method 1. In addition, compound (XI) can be also produced by reacting 1-(3-benzylthiopropyl)piperidin-4-ylmethylamine obtained by using compound (XIV) instead of compound (IX) in Method 4 with compound (IV) according to Method 3.

Method 7: A quaternary ammonium salt of the compound (I) can be synthesized by the following route.

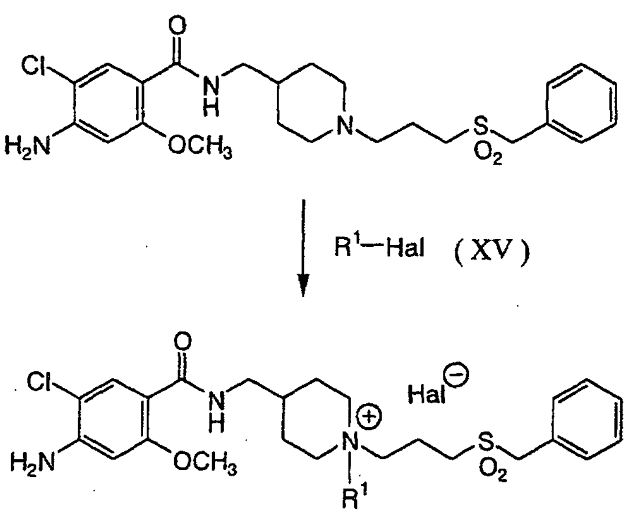


(I)

(I')

wherein $R^1$ is lower alkyl and other symbols are as defined above.

That is, compound (I') can be produced by reacting compound (I) with compound (XV) in a suitable solvent.

The solvent to be used includes, methylene chloride, 1,2-dichloroethane, chloroform, methanol, ethanol, propanol, isopropyl alcohol, butanol, tetrahydrofuran, diethyl ether, dioxane, benzene, toluene and xylene. While the reaction temperature varies depending on the kind of solvent to be used, it is generally 0°C to 40°C, and the reaction time which varies depending on reaction temperature is generally 1-24 hours.

The compound of the present invention thus obtained can be separated and purified from reaction mixture by a method known per se, such as recrystallization, column chromatography, and the like.

The compound of the present invention can be converted to acid addition salt by treating the compound with an acid such as hydrochloric acid, sulfuric acid, hydrobromic acid, phosphoric acid, nitric acid, methanesulfonic acid, ethanesulfonic acid, fumaric acid, maleic acid, benzoic acid, citric acid, malic acid, mandelic acid, p-toluenesulfonic acid, acetic acid, succinic acid, malonic acid, lactic acid, salicylic acid, gallic acid, picric acid, carbonic acid, ascorbic acid, trifluoroacetic acid and tartaric acid in a suitable solvent such as methanol, ethanol, water and a mixture thereof.

The compound of the present invention can be converted to N-oxide compound by oxidation using 1 to 10 equivalents, preferably from monoequivalent to a slightly excess, of an oxidizing agent such as m-chloroperbenzoic acid, perbenzoic acid, peracetic acid, trifluoroperacetic acid, permaleic acid, sodium bromite, sodium hypochlorite and hydrogen peroxide, in a suitable solvent such as chloroform, methylene chloride, tetrahydrofuran, dioxane, dimethylformamide, acetic acid, water and mixed solvents thereof, at -50°C to room temperature for 5 minutes to 24 hours. This oxidation reaction can be also carried out in the presence of a catalyst such as sodium tungstate.

When the crystals of the obtained compound of the present invention are anhydrides, the crystals can be converted to hydrates or solvates by a treatment with aqueous solvent or other solvent.

The compound of the present invention and pharmaceutically acceptable salts thereof have selective and high affinity for 5-$HT_4$ receptors, as well as activates them, and are useful as pharmaceutical agents for the prophylaxis and treatment of gastrointestinal diseases (e.g., reflux esophagitis; gastroesophageal reflux such as that accompanying cystic fibrosis; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis, diabetes, and the like; gastrointestinal disorders such as indigestion, meteorism, abdominal indefinite complaint, and the like; constipation such as atonic constipation, chronic constipation, and constipation caused by spinal cord injury, pelvic diaphragm failure and the like; and irritable bowel syndrome); central nervous system disorders (e.g., schizophrenia, depression, anxiety, disturbance of memory and dementia); cardiac function disorders (e.g., heart failure and myocardial ischemia); and urinary diseases (e.g., dysuria caused by urinary obstruction, ureterolith, prostatomegaly, spinal cord injury, pelvic diaphragm failure, etc.) and the like. In addition, since they possess anti-nociceptive action, they are useful as anti-nociceptive agents for pain relief which increase the threshold value of pain.

The compounds of the present invention and pharmaceutically acceptable salts thereof can be administered orally or parenterally by inhalation, rectal administration or local administration. They can be used as pharmaceutical compositions or pharmaceutical preparations such as powders, granules, tablets, pills, capsules, injections, syrups, emulsions, elixirs, suspensions and solutions, wherein at least one inventive compound is used solely or in combination with pharmaceutically acceptable carriers (e.g., excipients, binders, disintegrators, correctives, corrigents, emulsifying agents, diluents and/or solubilizers).

The pharmaceutical composition can be formulated into a pharmaceutical preparation by a conventional method. In the present specification, "parenteral" includes subcutaneous injection, intravenous injection, intramuscular injection, intraperitoneal injection and transfusion. The preparation for injection, such as a sterile aqueous or oily suspension for injection, can be prepared using a suitable dispersing agent or a wetting agent and a suspending agent, according to a method known in the pertinent field. The sterile preparation for injection may be a sterile injectable solution or suspension in a non-toxic diluent or solvent permitting parenteral administration, such as an aqueous solution. Examples of the vehicle and solvent which can be used include water, Ringer solution, isotonic saline and the like. In addition, sterile nonvolatile oil can be generally used as a solvent or a solvent for suspension. For this end, any nonvolatile oil or fatty acid can be used, inclusive of natural, synthetic or semi-synthetic fatty oil or fatty acid, and natural, synthetic or semi-synthetic mono-, di- or triglycerides. The suppository for rectal administration can be produced by mixing the drug with suitable non-irritative excipient, such as cocoa butter and polyethylene glycols which are solid at ordinary temperature, liquid at the temperature of the intestinal tract and melted in the rectum to release the drug. The solid dosage form for oral administration includes the above-mentioned ones such as powders, granules, tablets, pills, capsules and the like. In these dosage form, the active ingredient is admixed with at least one additive such as sucrose, lactose, cellulose sugar, mannitol, maltitol, dextran, starches, agar, arginates, chitins, chitosans, pectins, tragacanth gums, gum arabic, gelatins, collagens, casein, albumin, synthetic or semi-synthetic polymers and glycerides. In these dosage forms, routine additives can be added, which may be inert diluents, lubricants such as magnesium stearate, preservatives such as p-hydroxybenzoate, sorbic acid, antioxidants such as ascorbic acid, α-tocopherol and cysteine,

disintegrators, binders, tackifiers, buffers, sweeteners, flavors, perfumes and the like. An enteric coating may be applied to tablets and pills. The liquid agents for oral administration may be pharmaceutically acceptable emulsions, syrups, elixirs, suspensions, solutions and the like, which may contain inert diluents (e.g., water), generally used in the pertinent field.

While the dose for a certain patient is determined according to age, body weight, general health conditions, sex, diet, administration time, administration route, clearance rate, combination of drugs, the state of the disease for which the patient is then undergoing treatments, and other factors. The compounds of the present invention and pharmaceutically acceptable salts thereof show low toxicity and can be used safely. While the daily dose varies depending on the condition and body weight of the patient, the kind of compound, administration route and the like, it is, for example, about 0.01-50 mg/person/day, preferably 0.01-20 mg/person/day, for parenteral administration by a subcutaneous, intravenous, intramuscular or intrarectal route, and about 0.01-150 mg/person/day, preferably 0.1-100 mg/person/day, for oral administration.

Best Mode for Embodying the Invention

The present invention is specifically described by preparation examples, working examples and formulation examples, to which the present invention is not limited. In the formulas, Boc means tert-butoxycarbonyl.

## Preparation Example 1

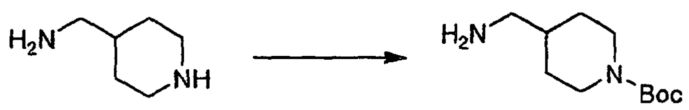

4-(Aminomethyl)piperidine (137 g) was dissolved in toluene (1200 ml), benzaldehyde (127 g) was added thereto, and the mixture was stirred at refluxing temperature for 6.5 hours. The reaction mixture was cooled to room temperature, and di-tert-butyl dicarbonate (288 g) was dropwise added thereto, and the mixture was stirred at room temperature for 1 hour. The reaction mixture was concentrated under reduced pressure, and IN potassium hydrogensulfate (1200 ml) was added to the obtained residue, which was followed by stirring at room temperature for 4 hours. The reaction mixture was washed 3 times with isopropyl ether and made alkaline with 10% sodium hydroxide solution. The mixture was extracted with chloroform. The organic layer was washed with a saturated aqueous sodium chloride solution, dried over sodium sulfate and concentrated under reduced pressure to give 236.5 g of 4-aminomethyl-1-(tert-butoxycarbonyl)piperidine.

$^1$H-NMR ($CDCl_3$, ppm)$\delta$ :1.01-1.30(5H,m), 1.45(9H,s), 1.65-1.75(2H,m), 4.07-4.16(2H,m)

## Preparation Example 2

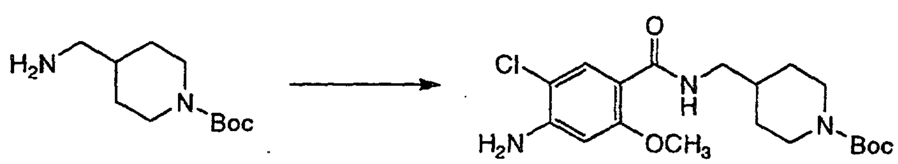

To a solution of 4-aminomethyl-1-(tert-butoxycarbonyl)piperidine (236.5 g) in dimethylformamide (1200 ml) were added 4-amino-5-chloro-2-methoxybenzoic acid (130 g) and 1-hydroxybenzotriazole (91.4 g). 1-Ethyl-3-(3-dimethylaminopropyl)carbodiimide hydrochloride (129.6 g) was added thereto under ice-cooling, and the mixture was stirred at room temperature for 1 hour. The reaction mixture was concentrated under reduced pressure, and water was added to the obtained residue, which was followed by extraction with chloroform. The organic layer was washed successively with 10% aqueous potassium carbonate solution and a saturated aqueous sodium chloride solution and dried. The solvent was evaporated under reduced pressure and the obtained residue was purified by silica gel column chromatography (chloroform) to give 272 g of 4-amino-5-chloro-2-methoxy-N-(1-(tert-butoxycarbonyl)piperidin-4-ylmethyl) benzamide.

$^1$H-NMR ($CDCl_3$, ppm)δ :1.10-1.27(2H,m), 1.45(9H,s), 1.65-1.85(3H,m), 2.62- 2.75(2H,m), 3.27-3.37(2H,m), 3.88(3H,s), 4.61(2H,s), 6.34(1H,s), 7.72-7.82(1H,m), 8.07(1H,s)

## Preparation Example 3

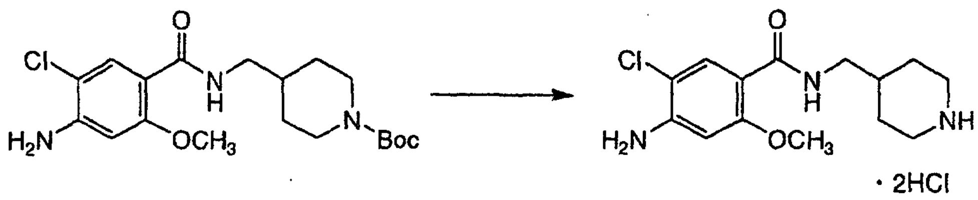

4.25 M Hydrochloric acid-dioxane (1000 ml) was dropwise added to a solution (600 ml) of 4-amino-5-chloro-2-methoxy-N-(1-(tert-butoxycarbonyl)piperidin-4-ylmethyl)benzamide (272 g) in dioxane, and the mixture was stirred at room temperature for 2 hours. The precipitated crystals were collected by filtration and recrystallized from methanol-isopropyl ether to give 211 g of 4-amino-5-chloro-2-methoxy-N-(piperidin-4-ylmethyl)benzamide dihydrochloride. m.p. 235-239°C

## Preparation Example 4

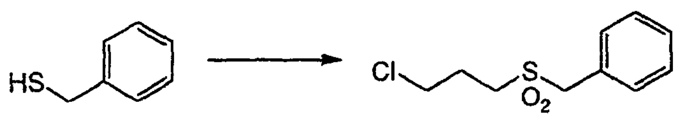

Benzyl mercaptan (1.5 kg) and potassium carbonate (2.5 kg) were suspended in methanol (15 liters) under a nitrogen stream, and 1-bromo-3-chloropropane (3.0 kg) was added thereto at 0-3°C over 10 minutes. The mixture was stirred at 0-10°C for 0.5 hours and then at 10-25°C for 2 hours. The reaction mixture was concentrated under reduced pressure, water was added thereto and the mixture was extracted with ethyl acetate. The organic layer was washed with water and the solvent was evaporated under reduced pressure. The residue was dissolved in formic acid (12 liters), 30% aqueous hydrogen peroxide solution (2.73 liters) was dropwise added thereto at 4-18°C over 30 minutes and the mixture was stirred at 18-45°C for 2 hours. Water was added to the reaction mixture and the precipitated crystals were collected by filtration. The crystals were washed with water and dried to give 3-benzylsulfonylpropyl chloride (2.55 kg) as crystals, melting point 110-111°C.
IR (KBr, $cm^{-1}$): 1117
$^1$H-NMR($CDCl_3$)δ(ppm): 2.22-2.30 (2H,m), 3.02 (2H, t, J=7.6Hz), 3.63 (2H, t, J=6.3Hz), 4.25 (2H, s), 7.42 (5H, s)

## Preparation Example 5

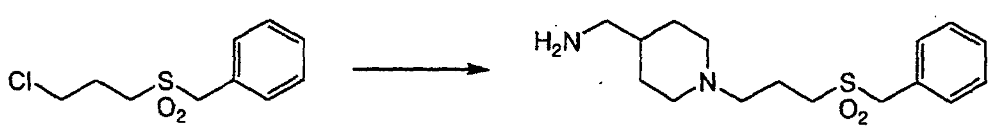

4-Aminomethylpiperidine (1.7 kg) and benzaldehyde (1.6 kg) were dissolved in toluene (13 liters) and the mixture was refluxed under heating for 6 hours. The reaction mixture was concentrated under reduced pressure and the residue was dissolved in acetonitrile (40 liters). Thereto were added 3-benzylsulfonylpropyl chloride (4.5 kg), potassium carbonate (2.7 kg) and potassium iodide (250 g), and the mixture was refluxed under heating for 6 hours. After cooling, water was added thereto, the mixture was extracted with toluene and the solvent was evaporated under reduced pressure. Toluene (30 liters) and water (30 liters) were added to the residue, concentrated hydrochloric acid (3.2 liters) was added thereto under water-cooling and the mixture was stirred for 3.5 hours. The mixture was filtered through celite and insoluble matter was filtered off. The aqueous layer was adjusted to pH 7 with potassium carbonate,

and the mixture was washed with chloroform. The aqueous layer was saturated with potassium carbonate and extracted with chloroform. The organic layer was dried over sodium sulfate, and the solvent was evaporated under reduced pressure, which was followed by crystallization from diisopropyl ether to give 1-(3-benzylsulfonylpropyl)piperidin-4-ylmethylamine (4.18 kg) as crystals, melting point 130-134°C (half-melting at 60°C).
IR(KBr, $cm^{-1}$): 2929, 1313, 1274, 1130
$^1$H-NMR($CDCl_3$)δ(ppm): 1.08-1.36 (3H, m), 1.69 (2H, dd, J=11.9Hz), 1.87-2.00 (4H, m), 2.38 (2H, t, J=6.9Hz), 2.56 (2H, d, J=5.9Hz), 2.81-2.94 (4H, m), 4.23 (2H, s), 7.38-7.43 (5H, m)

## Working Example 1

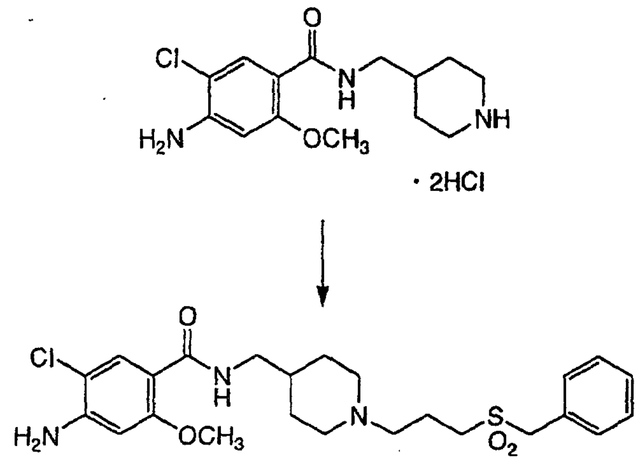

To a solution (250 ml) of 4-amino-5-chloro-2-methoxy-N-(piperidin-4-ylmethyl)benzamide dihydrochloride (8.00 g) in dimethylformamide, potassium carbonate (13.4 g) and 3-benzylsulfonylpropyl chloride (6.03 g) were added and the mixture was stirred at 70-80°C for 7.5 hours. Water was added to the reaction mixture and the mixture was extracted with chloroform. The organic layer was washed with a saturated aqueous sodium chloride solution and dried. The solvent was evaporated under reduced pressure, and the obtained residue was purified by silica gel column chromatography and recrystallized from ethanol to give 4-amino-5-chloro-2-methoxy-N-((1-(3-benzylsulfonylpropyl)piperidin-4-yl)methyl)benzamide (Y-36912; 4.40 g), melting point 170-171°C.

## Working Example 2

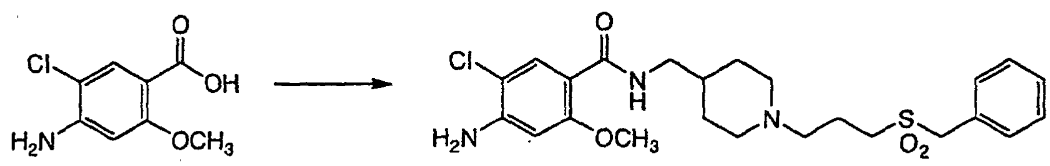

4-Amino-5-chloro-2-methoxybenzoic acid (2.1 kg) and triethylamine (2.3 kg) were dissolved in chloroform (70 liters) and thereto was dropwise added isobutyl chloroformate (1.4 kg) at -4°C to -2°C. The mixture was stirred at said temperature for 1 hour, thereto was added 1-(3-benzylsulfonylpropyl)piperidin-4-ylmethylamine (3.9 kg) at -4°C to -3°C and the mixture was stirred for 1 hour. The reaction mixture was washed with 5% hydrochloric acid (12 liters), a 5% aqueous sodium hydrogencarbonate solution and water in order, and the solvent was evaporated. The residue was dissolved in ethanol (200 liters) under heating. The solution was decolorized with active-carbon and concentrated to about 100 liters.
Diisopropyl ether (100 liters) was added thereto and the precipitated crystals were collected by filtration to give crude crystals (4.28 kg). The crude crystals were recrystallized from ethanol (170 liters), and the obtained crystals were dried and pulverized with atomizer to give 4-amino-5-chloro-2-methoxy-N-((1-(3-benzylsulfonylpropyl)piperidin-4-yl)methyl)benzamide (Y-36912; 3.88 kg) as pale yellowish white crystals, melting point 170-171°C.

IR (KBr, $cm^{-1}$): 3466, 3396, 3303, 3187, 2916, 1639, 1589, 1300, 1120
$^1H$-NMR ($CDCl_3$) δ (ppm): 1.17-1.32 (2H, m), 1.50-1.63 (1H, m), 1.69 (2H, bd, J=13.2Hz), 1.86-2.00 (4H, m), 2.37 (2H, t, J=6.9Hz), 2.82 (2H, bd, J=11.9Hz), 2.88-2.94 (2H, m),3.31 (2H, t, J=6.3Hz), 3.88 (3H, s), 4.22 (2H, s), 6.30 (1H, s), 7.37-7.43 (5H, m), 7.73 (1H, t, J=5.6Hz), 8.10 (1H, s)

| elemental analysis | | | |
|---|---|---|---|
| Calculated | C, 58.34; | H, 6.53; | N, 8.51 |
| Found | C, 58.15; | H, 6.56; | N, 8.49 |

The formulation example of the compound of the present invention as a medicament is given in the following.

| Formulation example | (mg) |
|---|---|
| Compound of the present invention | 10.0 |
| Lactose | 109.6 |
| Microcrystalline cellulose | 27.4 |
| Light anhydrous silicic acid | 1.5 |
| Magnesium stearate | 1.5 |
| | 150 (per tablet) |

The compound of the present invention (30 g), lactose (328.8 g) and microcrystalline cellulose (82.2 g) are mixed. The mixture-is compressed using a roller compactor to give flake-like compression product. Using a hammer mill, the flake-like compression product is pulverized. The pulverized product is passed through a 20-mesh sieve. Light anhydrous silicic acid (4.5 g) and magnesium stearate (4.5 g) are added and mixed. The mixture is punched with a 7.5 mm diameter pounder to give 3,000 tablets weighing 150 mg per tablet.
The bioactivity of the compounds of the present invention and pharmaceutically acceptable salts thereof is explained by way of Experimental Examples.

Experimental Example 1 : 5-$HT_4$ receptor binding test

According to the method of C.J. Grossman et al. [British Journal of Pharmacology, vol. 109, pp. 618-624 (1993)1, a 5-$HT_4$ receptor binding test was run using, as a tracer ligand, 1-methyl-1H-indole-3-carboxylic acid 1-(2-methylsulfonylaminoethyl)-piperidin-4-ylmethyl ester substituted by tritium (hereinafter to be referred to as [$^3$H]GR113808). Crude synapse membrane sample was prepared from guinea pig striatum, suspended in 50 mM HEPES buffer (pH 7.4) and used for testing. Y-36912 having several different concentrations and [$^3$H]GR113808 (final concentration 0.1 nM) were added to this suspension and allowed to react at 37°C. Thirty minutes later, the reaction mixture was filtered by suction with cell harvester. The filter was washed with 50 mM HEPES buffer, and the radioactivity on the filter was counted by liquid scintillation counter. The non-specific binding was determined in the presence of 1μM GR113808. The concentration of Y-36912 necessary for 50% inhibition ($IC_{50}$) was determined from graph and converted to binding inhibition constant (Ki value). The result is shown in Table 1.

Experimental Example 2 : 5-$HT_3$ receptor binding test

According to the method of Nelson and Thomas [Biochemical Pharmacology, vol. 38, pp. 1693-1695 (1989)], a 5-$HT_3$ receptor binding test was run using, as a tracer ligand, endo-l-methyl-N-(9-methyl-9-azabicyclo[3.3.1]non-3-yl)-1H-indazole-3-carboxamide substituted by tritium (hereinafter to be referred to as [$^3$H]granisetron). Crude synapse membrane sample was prepared from rat cerebral cortex, suspended in 50 mM HEPES buffer (pH 7.4) and used for testing. Y-36912 having several different concentrations and [$^3$H]granisetron (final concentration 1.0 nM) were added to this suspension and allowed to react at 25°C. Thirty minutes later, the reaction mixture was filtered by suction with cell harvester. The filter was washed with 50 mM Tris buffer (pH 7.4), and the radioactivity on the filter was counted using liquid scintillation counter or β-plate. The non-specific binding was determined in the presence of 1 μM tropisetron. The concentration of Y-36912 necessary for 50% inhibition ($IC_{50}$) was determined from graph and converted to binding inhibition constant (Ki value). The result is shown in Table 1, wherein * shows $IC_{50}$.

Table 1

| | Affinity for receptor (Ki value, nM) | |
|---|---|---|
| | $5\text{-}HT_4$ | $5\text{-}HT_3$ |
| Y-36912 | 1.5 | >1000* |

Experimental Example 3: contraction of the isolated guinea pig ascending colon

Male Hartley guinea pig was killed by a blow, ascending colon was taken from it and longitudinal muscle was separated. About 3 cm longitudinal muscle segment was suspended in 10 ml of a Tyrode solution with the load of 1 g. Tyrode solution contained methysergide (1 μM) and granisetron (1 μM) to inhibit responses mediated by $5\text{-}HT_1$, $5\text{-}HT_2$ and $5\text{-}HT_3$ receptors. Tyrode solution was maintained at 37°C and ventilated with a mixed gas of $O_2$ (95%) and $CO_2$ (5%). The contraction was isotonically measured via isotonic transducer. After an equilibrium period of about 30 minutes, the contraction by methacholine (30 μM) was measured two times at 15 minute intervals. Twenty minutes later, Y-36912 was added into the bath and the contraction was measured in the presence of a solvent or 1-methyl-1H-indole-3-carboxylic acid • 1-(2-methylsulfonylaminoethyl)-pyperidin-4-ylmethyl ester (hereinafter to be referred to as GR113808, 10nM), which is $5\text{-}HT_4$ receptor antagonist. The time of pretreatment with the solvent or GR113808 was 10 minutes. To avoid tachyphylaxis, the contraction induced by one concentration per preparation was measured. The contraction in the presence of a solvent or GR113803 was measured using a preparation from the same animal. The contraction effect of Y-36912 was measured as a percentage of responses to the second contraction by methacholine. The $EC_{50}$ value, the concentration causing 50 % of the contraction, was determined by linear regression analysis. The result is shown in Table 2. Antagonism of GR113808 to contraction of Y-36912 was calculated as $pK_B$ according to the following equation. $pK_B$ value was 8.8. $pK_B$= logic (concentration ratio - 1) - $\log_{10}$ (molar concentration of GR113808)

Table 2

| | Contraction effect on the isolated ascending colon ($EC_{50}$ value, nM) |
|---|---|
| Y-36912 | 10.8 |

Experimental Example 4 : promotion of postprandial gastrointestinal motility in dogs without anesthesia

According to the method of Ito et al. [Journal of Smooth Muscle Research, vol. 13, pp. 33-43 (1976)], male and female adult mongrel dogs were subjected to laparotomy under pentobarbital anesthesia, and force transducer was sutured to the serous membrane of various sites of gastrointestinal tract in the direction of circular muscle. After about two weeks' recovery period, postprandial gastrointestinal motility was measured in vigilance. The results are shown in Table 3 as the difference in motility index (g·min) between before and after administration of Y-36912. Y-36912 was administered intravenously.

Table 3

| Compound | mg/kg i.v. | N | Difference of motility index (g·min) | |
|---|---|---|---|---|
| | | | Stomach | Colon |
| Vehicle | | 4 | -8.6 ± 3.5 | -2.6 ± 18.5 |
| Y-36912 | 0.01 | 4 | 41.3 ± 35.4 | 27.1 ± 6.8 |
| | 0.03 | 4 | 65.1 ± 38.1** | 28.3 ± 8.5 |
| | 0.1 | 4 | 60.5 ± 18.1** | 58.2 ± 11.9* |

Mean ± S.E.,
*; P<0.05,
**; P<0.01

As shown in the above Table, Y-36912 increased gastric (upper gastrointestinal) motility at the doses of 0.03 mg/kg or above and colonic (lower gastrointestinal) motility at the dose of 0.1 mg/kg. Experimental Example 5 : evaluation of bioavailability in rats

Female SD rats (4 per group) were administered with 3 mg/kg (i.v.) and 30 mg/kg (p.o.) of Y-36912. The blood was taken with the passage of time, and the concentration of the drug in plasma was measured with high performance

liquid chromatography. The bioavailability was calculated from the following formula, and found to be 85.2% and fine for Y-36912. In the formula, AUC means area under the plasma concentration-time curve.

$$\frac{\text{AUC by oral administration}}{\text{AUC by intravenous administration}} \times \frac{\text{Dose of intravenous administration}}{\text{Dose of oral administration}} \times 100(\%)$$

Experimental Example 6 : measurement of absorption by small intestine in rat

Male SD rats (3 per group) fasted overnight were subjected to laparotomy under ether anesthesia, and the upper part of small intestine was ligated to form a loop (15-20 cm in length). The drug was dissolved in 5% glucose to the drug concentration of 5 mg/10 ml, and 10 ml/kg thereof was injected into the loop. At 2 hours from the drug injection, the loop was removed, washed with 100 ml of 0.1 N hydrochloric acid-methanol (3:7), and the drug concentration was determined by high performance liquid chromatography. The absorption ratio was calculated according to the following formula. The absorption of Y-36912 was 60.5±13.7 %

$$\frac{\text{Injected amount of drug (mg) - drug concentration in loop (mg/ml) x 100 (ml)}}{\text{Injected amount of drug (mg)}} \times 100\ (\%)$$

Experimental Example 7: stimulation of defecation in mice

Male ICR mice were subcutaneously administered with Y-36912 after adaptation to experimental surroundings for thirty minutes. The number and wet weight of feces excreted in 2 hours from immediately after administration were measured. The results are shown in Table 4.

Table 4

| Compound | mg/kg s.c | N | Number | Promotion(%) | Wet weight | Promotion(%) |
|---|---|---|---|---|---|---|
| Vehicle | - | | 3.4 ± 0.4 | | 56.5 ± 7.9 | |
| Y-36912 | 0.03 | 20 | 5.4 ± 0.7 | 59 | 78.2 ± 8.6 | 38 |
| | 0.1 | 20 | 6.5 ± 0.8** | 91 | 95.7 ± 10.6* | 69 |
| | 0.3 | 20 | 6.2 ± 0.8* | 82 | 94.7 ± 10.3* | 68 |
| | 1 | 20 | 5.4 ± 0.5 | 59 | 97.7 ± 12.5* | 73 |

Mean ± S.E.,
*; P<0.05,
** P<0.01

As shown in the above Table, Y-36912 showed significant promotion of defecation by administration at the doses of 0.1 mg/kg or above.

Experimental Example 8 : Promotion of transit in large intestine in guinea pig

Male Hartley guinea pigs were subjected to laparotomy under ether anesthesia, and a silicone tube was inserted into the beginning of ascending colon and fixed there. The other end was passed under the skin to be exposed from the back and sutured. At day 5 postoperation, Y-36912 was administered orally and Evans blue solution was injected into the colon through the silicon tube. One hour later, the large intestine was removed, and the entire length of the large intestine and the length of the transit of the Evans blue solution were measured. Evans blue transit ratio relative to the entire length of the large intestine (100 %) was calculated. The results are shown in Table 5.

Table 5

| Compound | mg/kg p.o. | N | Ratio of transit in large intestine (%) | Promotion (%) |
|---|---|---|---|---|
| Vehicle | | 16 | 52.4 ± 5.2 | |

Table 5 (continued)

| Compound | mg/kg p.o. | N | Ratio of transit in large intestine (%) | Promotion (%) |
|---|---|---|---|---|
| Y-36912 | 0.1 | 16 | 57.0 ± 7.0 | 9 |
| | 0.3 | 16 | 70.9 ± 7.9 | 35 |
| | 1 | 17 | 77.2 ± 6.4* | 47 |
| | 3 | 17 | 80.1 ± 5.8* | 53 |

Mean ± S.E.,
*; P<0.05

As shown in the above Table, Y-36912 significantly accelerated transit in large intestine in guinea pigs at the doses of 1 mg/kg or above.

Experimental Example 9 : promotion of gastric emptying in rats

Barium pellets (ca. 1 mm diameter) coated with polystyrene was intragastrically administered to female Wistar rats. One hour later, stomach was removed, and the remaining pellets were counted. Y-36912 was orally administered one hour before administration of the pellets. The results are shown in Table 6.

Table 6

| Compound | mg/kg p.o. | N | Number of pellets | Promotion (%) |
|---|---|---|---|---|
| Vehicle | - | 16 | 28.1 ± 2.0 | |
| Y-36912 | 0.1 | 16 | 21.1 ± 2.8 | 25 |
| | 0.3 | 16 | 18.8 ± 3.0* | 33 |
| | 1 | 16 | 19.5 ± 3.1 | 31 |
| | 3 | 16 | 13.4 ± 1.8** | 52 |

Mean ± S.E.,
*; P<0.05,
**; P<0.01

As shown in the above Table, Y-36912 significantly accelerated gastric emptying at the doses of 0.3 mg/kg and 3 mg/kg.

Experimental Example 10: Toxicity testing

There was no death when four female SD rats were administered orally with Y-36912 at a dose of 750 mg/kg. Female and male SD rats were administered repeatedly with Y-36912 at a dose of 30 mg/kg for 10 days, but no animal died and there were no abnormal signs related to this compound, general condition, body weight, food consumption, hematological examination, blood biochemical analysis, urinanalysis, organ weight or histopathological examination. When beagle dogs (LRE-strain) were administered repeatedly with Y-36912 at a dose of 3 mg/kg for 10 days, there were no abnormal signs related to this compound.

Industrial Applicability

From various pharmacological testings inclusive of the above-mentioned Experimental Examples and toxicity testing, it is evident that the compound of the present invention and pharmaceutically acceptable salts thereof have selective and high affinity for 5-$HT_4$ receptors, activate them, and are useful as a medicament for the prophylaxis and treatment of gastrointestinal diseases, central nervous system disorders, cardiac function disorders, urinary diseases and the like. They have less toxicity and show superior absorption.

**Claims**

**1.** 4-Amino-5-chloro-2-methoxy-N-((1-(3-benzylsulfonylpropyl)piperidin-4-yl)methyl)benzamide, a pharmaceutically

acceptable salt thereof or an N-oxide compound thereof.

2. A pharmaceutical composition comprising 4-amino-5-chloro-2-methoxy-N-((1-(3-benzylsulfonylpropyl)piperidin-4-yl)methyl)benzamide, a pharmaceutically acceptable salt thereof or an N-oxide compound thereof, and a pharmaceutically acceptable additive.

3. A serotonin 4 receptor agonist comprising 4-amino-5-chloro-2-methoxy-N-((1-(3-benzylsulfonylpropyl)piperidin-4-yl)methyl)benzamide, a pharmaceutically acceptable salt thereof or an N-oxide compound thereof as an active ingredient.

4. A gastrointestinal prokinetic agent comprising 4-amino-5-chloro-2-methoxy-N-((1-(3-benzylsulfonylpropyl)piperidin-4-yl)methyl)benzamide, a pharmaceutically acceptable salt thereof or an N-oxide compound thereof as an active ingredient.

5. A prophylactic or therapeutic agent for gastrointestinal diseases selected from reflux esophagitis; gastroesophageal reflux; Barrett syndrome; intestinal pseudoileus; acute or chronic gastritis; gastric or duodenal ulcer; Crohn's disease; non-ulcer dyspepsia; ulcerative colitis; postgastrectomy syndrome; postoperative digestive function failure; delayed gastric emptying caused by gastric neurosis, gastroptosis and diabetes; gastrointestinal disorder which is indigestion, meteorism or abdominal indefinite complaint; constipation which is atonic constipation, chronic constipation, or constipation caused by spinal cord injury or pelvic diaphragm failure; and irritable bowel syndrome, which agent comprises 4-amino-5-chloro-2-methoxy-N-((1-(3-benzylsulfonylpropyl)piperidin-4-yl)methyl)benzamide, a pharmaceutically acceptable salt thereof or an N-oxide compound thereof as an active ingredient.

## Patentansprüche

1. 4-Amino-5-chlor-2-methoxy-N-((1-(3-benzylsulfonylpropyl)piperidin-4-yl)methyl)benzamid, ein pharmazeutisch annehmbares Salz davon oder eine N-Oxidverbindung davon.

2. Pharmazeutische Zusammensetzung umfassend 4-Amino-5-chlor-2-methoxy-N-((1-(3-benzylsulfonylpropyl)piperidin-4-yl)methyl)benzamid, ein pharmazeutisch annehmbares Salz davon oder eine N-Oxidverbindung davon und ein pharmazeutisch annehmbares Additiv.

3. Serotonin-4-Rezeptorantagonist umfassend 4-Amino-5-chlor-2-methoxy-N-((1-(3-benzylsulfonylpropyl)piperidin-4-yl)methyl)benzamid, ein pharmazeutisch annehmbares Salz davon oder eine N-Oxidverbindung davon als aktiver Bestandteil.

4. Prokinetisches Magen-Darm-Mittel umfassend 4-Amino-5-chlor-2-methoxy-N-((1-(3-benzylsulfonylpropyl)piperidin-4-yl)methyl)benzamid, ein pharmazeutisch annehmbares Salz davon oder eine N-Oxidverbindung davon als aktiver Bestandteil.

5. Prophylaktisches oder therapeutisches Mittel für Magen-Darm-Erkrankungen, die aus Refluxösophagitis, gastroösophagealem Reflux, Barrett-Syndrom, Pseudodarmverschluß, akuter oder chronischer Gastritis, Magen- oder Zwölffingerdarmgeschwür, Morbus Crohn, nicht-ulzerativer Dyspepsie, ulzerativer Colitis, Postgastrektomiesyndrom, postoperativem Versagen der Verdauungsfunktion, durch Magerineurose, Gastroptose und Diabetes verursachter, verzögerter Magenentleerung, einer Magen-Darm-Störung, bei der es sich um eine Verdauungsstörung, Meteorismus oder undefinierte Bauchbeschwerden handelt, einer Verstopfung, bei der es sich um eine atonische Verstopfung, chronische Verstopfung oder eine durch eine Rückenmarksverletzung oder einen Beckenzwerchfelldefekt verursachte Verstopfung handelt, und einem Colon irritabile ausgewählt sind, wobei das Mittel 4-Amino-5-chlor-2-methoxy-N-((1-(3-benzylsulfonylpropyl)piperidin-4-yl)methyl)benzamid, ein pharmazeutisch annehmbares Salz davon oder eine N-Oxidverbindung davon als aktiver Bestandteil umfaßt.

## Revendications

1. 4-amino-5-chloro-2-méthoxy-N-((1-(3-benzyl-sulfonylpropyl)pipéridin-4-yl)méthyl)benzamide, un sel pharmaceutiquement acceptable de celui-ci ou un composé N-oxyde de celui-ci.

2. Composition pharmaceutique comprenant le 4-amino-5-chloro-2-méthoxy-N-((1-(3-benzylsulfonylpropyl) pipéridin-4-yl)méthyl)benzamide, un sel pharmaceutiquement acceptable de celui-ci ou un composé N-oxyde de celui-ci et un additif pharmaceutiquement acceptable,

3. Agoniste du récepteur 4 de la sérotonine comprenant le 4-amino-5-chloro-2-méthoxy-N-((1-(3-benzylsulfonylpropyl)pipéridin-4-yl)méthyl)benzamide, un sel pharmaceutiquement acceptable de celui-ci ou un composé N-oxyde de celui-ci comme ingrédient actif.

4. Agent procinétique gastro-intestinal comprenant du 4-amino-5-chloro-2-méthoxy-N-((1-(3-benzylsulfonyl-propyl) pipéridin-4-yl)méthyl)benzamide, un sel pharmaceutiquement acceptable de celui-ci ou un composé N-oxyde de celui-ci comme ingrédient actif.

5. Agent prophylactique ou thérapeutique pour des affections gastro-intestinales sélectionnées parmi l'oesophagite par reflux gastro-oesophagien ; le reflux gastro-oesophagien ; le syndrome de Barrett ; l'iléus paralytique intestinal ; la gastrite aiguë ou chronique ; l'ulcère duodénal aigu ou chronique ; la maladie de Crohn ; la dyspepsie non ulcéreuse ; la rectocolite hémorragique ; le syndrome de postgastrectomie ; la défaillance de la fonction digestive postopératoire ; le retard de la vidange gastrique dû à une névrose gastrique ; la gastroptose et le diabète ; un trouble gastro-intestinal qui est une indigestion, un météorisme ou un symptôme abdominal indéterminé ; la constipation qui est une constipation atonique, une constipation chronique ou une constipation provoquée par une blessure de la moelle épinière ou une défaillance du diaphragme pelvien et le côlon irritable, lequel agent comprend le 4-amino-5-chloro-2-méthoxy-N-((1-(3-benzylsulfonylpropyl) pipéridin-4-yl)méthyl)benzamide, un sel pharmaceutiquement acceptable de celui-ci ou un composé N-oxyde de celui-ci comme ingrédient actif.